# EUROPEAN PATENT SPECIFICATION

(11) **EP 3 856 296 B1**
(45) Date of publication and mention of the grant of the patent: **02.12.2026**
(21) Application number: 19765491.6
(22) Date of filing: 12.09.2019
(51) Int. Cl.: A61M 5/32

(54) **MULTI-ROWS PRONGS GRABBER**
GREIFER MIT MEHRREIHIGEN ZACKEN
PRÉHENSEUR À DENTS MULTIRANGÉES

(30) Priority: 24.09.2018 US 201862735307 P; 08.11.2018 EP 18205269
(43) Date of publication of application: 04.08.2021
(73) Proprietor: SHL Medical AG, 6302 Zug (CH)
(72) Inventor: FROST, Jason, Boca Raton, Florida 33431 (US); STEFANOV, Slobodan, Deerfield Beach, Florida 33442 (US)
(86) International application number: PCT/EP2019/074297
(87) International publication number: WO 2020/064336

(56) References cited:
- EP-A1- 2 886 144
- EP-A1- 3 257 536
- WO-A1-2013/058697
- WO-A2-2010/146358
- US-A- 5 098 400

## Description

### TECHNICAL AREA

The present disclosure relates to a needle shield remover and in particular to a needle shield remover for a needle shield of a medicament delivery device that has at least two sets of needle shield gripping members arranged offset in relation to each other.

### BACKGROUND OF INVENTION

Today's medicament delivery devices may be complex and involve many different components. The physical features of each component and the way components are assembled together has a direct effect on how these components are going to interact with each other and on the success rate of the medicament delivery device in use.

One such area is the removal of cap on the medicament delivery device to pull the needle shield away from a medicament container, wherein the needle shield is initially attached to a medicament container (e.g. syringe) to cover and protect the injection member (e.g. needle) on the medicament container. A successful needle shield removal involves two parts, namely the interaction between the cap and a needle shield remover as well as that between the needle shield remover and the needle shield. The cap needs to firmly grip the needle shield remover which in turn needs to firmly grip the needle shield in order for the user to successfully remove the needle shield by pulling the cap away from the medicament delivery device.

Currently, one example of a needle shield remover includes prongs that extend radially inward and configured to claws into the needle shield when the user pulls the cap. Further example can be found in WO2013/058697, US5098400, EP3257536, EP2886144, and WO2010/146358. In order for the needle shield remover to firmly grip the needle shield during removal, it is important for the prongs to be able to extend as much radially inward as possible in order to ensure that the prongs will claws deep into the needle shield. However, allowing prongs to extend too much radially inward may risk colliding with the needle shield and preventing the entry of the needle shield into the needle shield remover during assembly. Thus, there is a need to maximise gripping force of the needle shield remover on the needle shield during removal while making sure that the prongs do not impede the assembly of the needle shield with the needle shield remover.

As for the assembly between the cap and the needle shield remover, it is important to ensure that the needle shield remover is not able to detach itself from the cap. It is equally important for the needle shield remover to not be able to rotate when assembled with the cap as the rotation may cause the needle shield to rotate which may in turn twist and damage the delivery member on the medicament container. Thus, there is a need to firmly couple the needle shield remover with the cap.

### BRIEF DESCRIPTION OF INVENTION

In the present disclosure, when the term "distal" is used, this refers to the direction pointing away from the dose delivery site. When the term "distal part/end" is used, this refers to the part/end of the delivery device, or the parts/ends of the members thereof, which under use of the medicament delivery device is/are located furthest away from the dose delivery site. Correspondingly, when the term "proximal" is used, this refers to the direction pointing to the dose delivery site. When the term "proximal part/end" is used, this refers to the part/end of the delivery device, or the parts/ends of the members thereof, which under use of the medicament delivery device is/are located closest to the dose delivery site.

Further, the term "longitudinal", with or without "axis", refers to a direction or an axis through the device or components thereof in the direction of the longest extension of the device or the component.

The term "lateral", with or without "axis", refers to a direction or an axis through the device or components thereof in the direction of the broadest extension of the device or the component. "Lateral" may also refer to a position to the side of a "longitudinally" elongated body.

In a similar manner, the terms "radial" or "transversal", with or without "axis", refers to a direction or an axis through the device or components thereof in a direction generally perpendicular to the longitudinal direction, e.g. "radially outward" would refer to a direction pointing away from the longitudinal axis.

Also, if nothing else is stated, in the following description wherein the mechanical structure of the device and the mechanical interconnection of its components is described, the device is in an initial non-activated or non-operated state.

In view of the foregoing, a general object of the present disclosure is to provide a needle shield remover for a cap of a medicament delivery device, which needle shield remover is easier to assemble.

These and other aspects of, and advantages with, the present disclosure will become apparent from the following detailed description of the invention and from the accompanying drawings.

The invention is specified by the independent claims. Preferred embodiments are defined in the dependent claims.

According to an aspect of the disclosure it is characterized by a needle shield remover for assembly with a cap of a medicament delivery device. The needle shield remover comprises a remover body having a proximal end and a distal end, at least one first shield gripping member, and at least one second shield gripping member. The first shield gripping member and the second shield gripping member configured to engage the needle shield. The first shield gripping member and the second shield gripping member are arranged offset in relation to each other both in the longitudinal direction and along the circumference surface of the remover body.

The remover body is substantially cylindrical and has a first circumference and a second circumference circling around different portions of the remover body, at least two of the first shield gripping members are distributed around the first circumference, at least two of the second shield gripping members are distributed around the second circumference.

The first and second shield gripping members are extending radially inwardly and towards the proximal end, the first and second shield gripping members are configured to engage a circumferential side surface, or the distal end of the needle shield.

The first and second shield gripping members have first openings between the remover body and first portions, the first and second shield gripping members have second openings between the remover body and second portions, the first opening is connected to the second opening, the first openings allow the first portion to flex radially outward.

The needle shield remover comprises a cap attachment structure that includes a plurality of cap fastening members extending radially outward from the remover body, wherein a cut-out exists between two neighbouring cap fastening members.

According to another embodiment of the present disclosure, only one of the first shield gripping member is placed along a first line, only one of the second shield gripping member is placed along a second line.

The first and second shield gripping members have first portions extending from the remover body toward the proximal end and second portions extending radially inward from the first portions and toward the proximal end for engaging the needle shield.

According to one embodiment of the present disclosure, the second portion of the first shield gripping member has a shape different from that of the second portion of the second shield gripping member. However, in another embodiment, the second portion of the first shield gripping member is shaped substantially identical from the second portion of the second shield gripping member.

According to another object of the present disclosure, the needle shield remover comprises at least one cap attachment opening on an outer surface of the remover body to be configured to engage a remover attachment member of the cap.

### BRIEF DESCRIPTION OF DRAWINGS

In the following detailed description of the present disclosure, reference will be made to the accompanying drawings, of which
- Fig. 1: a perspective view of a needle shield remover according to prior art.
- Fig. 2: a horizontal view of a needle shield remover according to the first embodiment of the present disclosure.
- Fig. 3: a close-up perspective view of a needle shield remover according to a first embodiment of the present disclosure.
- Fig. 4: a top perspective view of a needle shield remover according to a first embodiment of the present disclosure.
- Fig. 5: another close-up perspective view of a needle shield remover according to the first embodiment of the present disclosure.
- Fig. 6: a perspective view of a cap and the needle shield remover according to the first embodiment of the present disclosure.

### DETAILED DESCRIPTION OF THE INVENTION

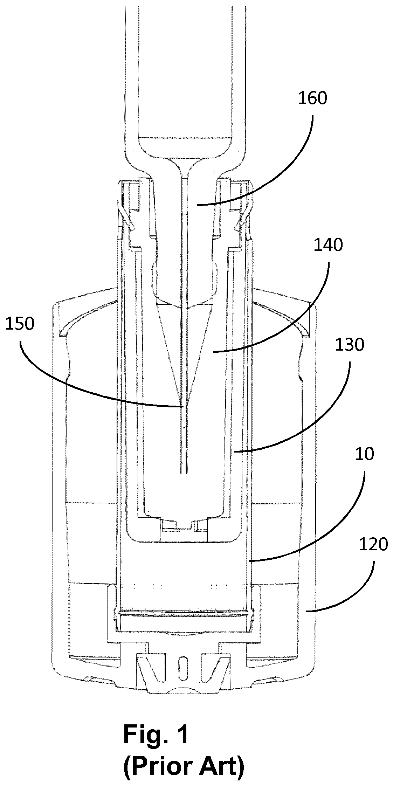

Fig. 1 shows a known needle shield remover 10 comprised in a cap 120. The cap 120 and the needle shield remover 10 are arranged on a rigid needle shield, or RNS 130, which covers a flexible needle shield, or FNS 140. A needle 150, of a syringe 160, is embedded in the FNS 140. The syringe 160 may be housed in a medicament delivery device (not shown), for instance an auto-injector. The needle shield remover 10 is attached to the cap 120 and comprises inwardly-projecting gripping members which engage the RNS 130, either by a distal end thereof or by engagement to a circumferential surface of the RNS 130, such that removal of the cap 120 pulls the RNS 130 and the FNS 140 away from the syringe to expose the needle 150.

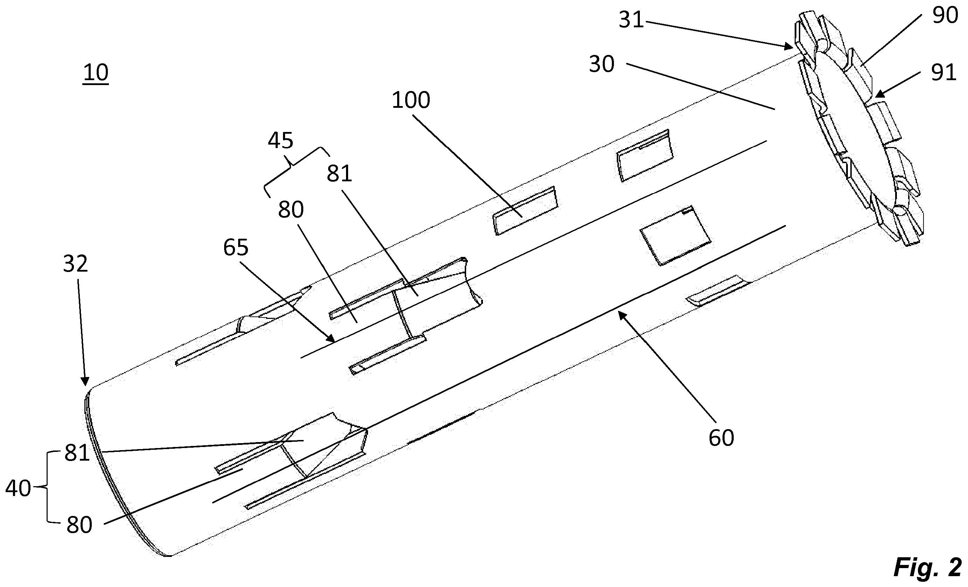

The present disclosure relates to a needle shield remover which is intended to be applied to known needle shields, for instance needle shields as shown in Fig. 1. The needle shield remover of the present disclosure is described in more detail in conjunction with Figs 2-6.

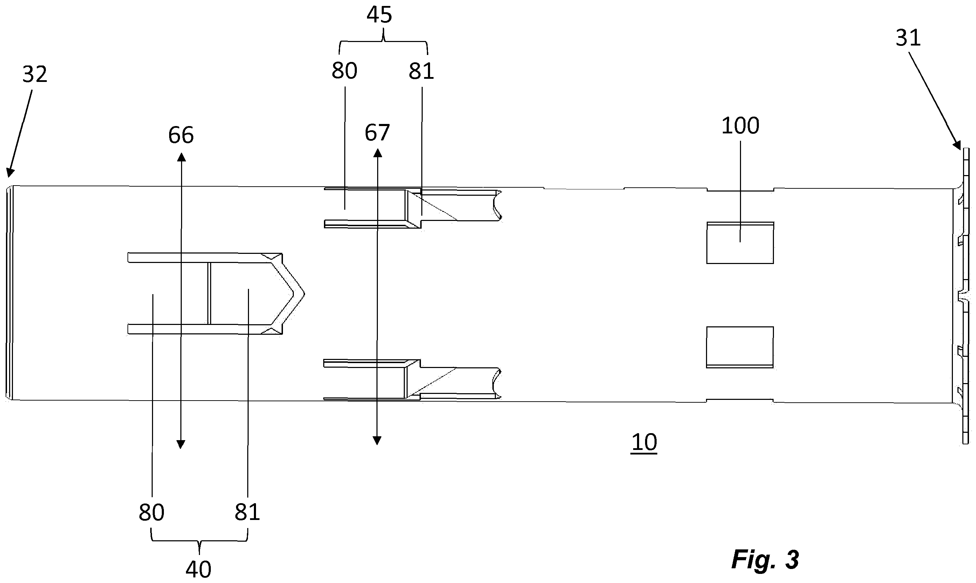

Fig. 2 shows a perspective view of the needle shield remover 10 according to a first embodiment of the present disclosure. Fig. 3 shows a horizontal view of a needle shield remover 10 according to the first embodiment of the present disclosure. Please refer to both Fig. 2 and Fig. 3 for the description that follows. The needle shield remover 10 comprises a longitudinally elongated tubular remover body 30 having a proximal end 31 and a distal end 32. The needle shield remover 10 also includes several first shield gripping members 40 and second shield gripping members 45 disposed on the remover body 30 and configured to make contact with the needle shield on a medicament container (such as syringe) to cover and protect the delivery member (such as needle) attached to the medicament container. The shield gripping members 40, 45 are resilient, extending radially inwardly and towards the proximal end 31. The needle shield remover 10 also includes a plurality of cap fastening members 90 and cut-outs 91 disposed on the proximal end 31 that are configured to couple the needle shield remover 10 with a cap which is then coupled to a medicament delivery device. The interaction between the needle shield remover 10 and the cap will be further explained later in the description below.

Each of the first shield gripping members 40 and second shield gripping members 45 include a first portion 80 and a second portion 81. The first portion 80 extends from the remover body 30 toward the proximal end 31. In other words, one end of the first portion 80 is connected to the remover body 30 while the other end extends towards the proximal end 31. On the other hand, one end of the second portion 81 is connected to the first portion 80 while the other end extends both toward the proximal end 31 and inwardly in order to make contact with the needle shield positioned within the remover body 30. The connection between the first portion 80 and second portion 81 allows the second portion 81 to be flexible with respect to the first portion 80. Similarly, the connection between the first portion 80 and the remover body 30 allows the first portion 80 to be flexible with respect to the remover body 30.

As illustrated in Fig. 2, the first shield gripping members 40 and second shield gripping members 45 are arranged offset in relation to each other both in the longitudinal direction and along the circumference surface of the remover body 30. As illustrated in Fig. 2, the first shield gripping member 40 extends along a first virtual line 60 while the second shield gripping member 45 extends along a second virtual line 65. As illustrated in Fig. 3, the first shield gripping members 40 are distributed around a first circumference 66 while the second shield gripping members 45 are distributed around a second circumference 67. Thus, it can be said that the first shield gripping members 40 and second shield gripping 45 are distributed on the remover body 30 in a zig-zag formation.

The above-mentioned configuration allows the needle shield remover 10 to distribute the gripping forces of shield gripping members 40, 45 to cover a greater surface area of the needle shield. By gripping different sections of the needle shield to spread out the gripping forces, the shield gripping members 40, 45 improve the overall gripping forces applied on the needle shield. This configuration allows the needle shield remover 10 to have greater overall gripping forces on the needle shield without needing to have excessive number of shield gripping members 40, 45 to occupy the entire remover body 30 or have bigger shield gripping members that may prevent the needle shield from being inserted in the needle shield remover 10 during assembly.

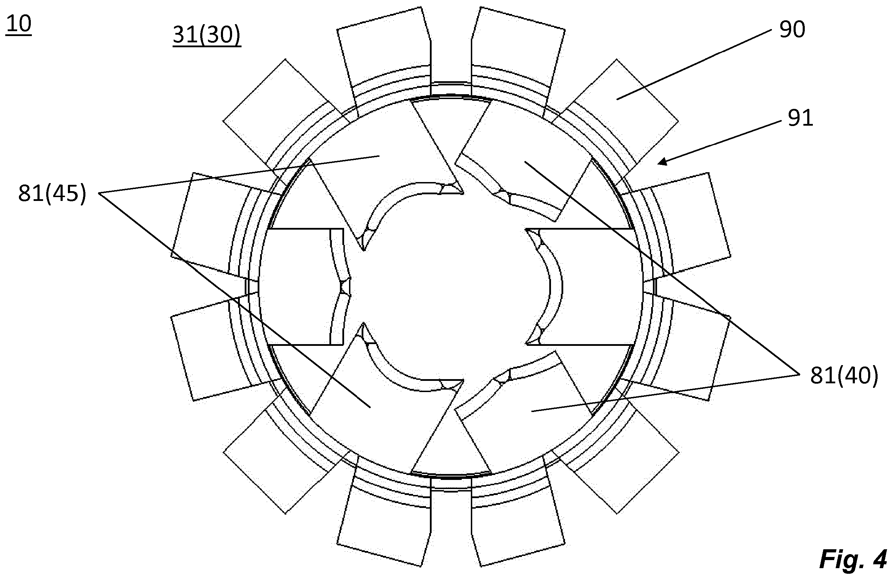

Here please refer to Fig. 4 that illustrates the top perspective view of the needle shield remover 10. As illustrated, the second portions 81 of the first shield gripping members 40 are configured to have pointed ends while those of the second shield gripping members 45 are configured to have curved ends. The above mentioned shapes of the second portions 81 are designed to create edges that allow the second portions 81 to dig deep into the needle shield during removal when the cap is pulled away from the medicament delivery device. In the present embodiment, the second portions 81 of the first and second shield gripping members 40, 45 are shaped differently, but they are not limited thereto. In other embodiments, the ends of the second shield gripping members 40, 45 can be configured to have substantially identical shape. Further, the second portions may also be configured to be rounded, angled, etc. depending on the gripping force required to grip the needle shield.

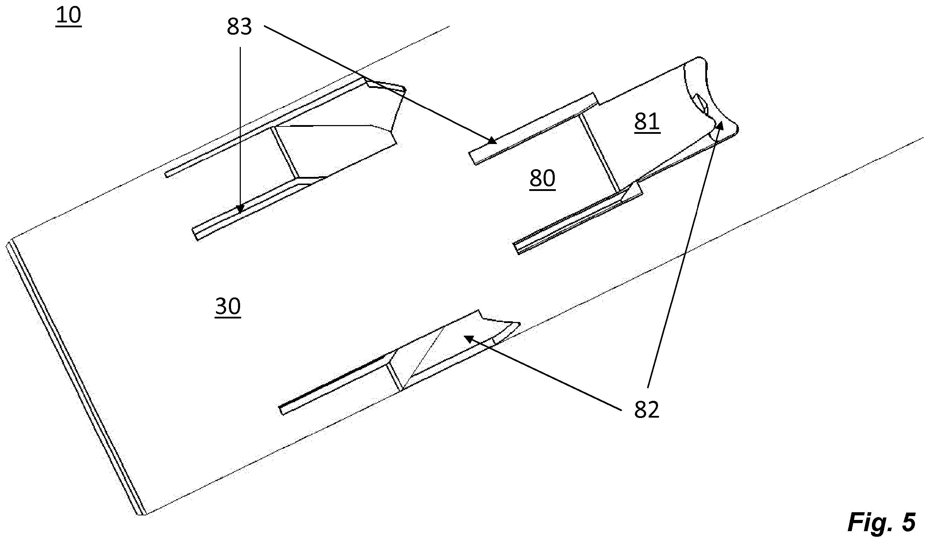

Fig. 5 shows a close up perspective view of a needle shield remover 10 according to a first embodiment of the present disclosure. In the present embodiment, the first shield gripping members 40 and second shield gripping members 45 are configured as cut-outs, or tongues, formed out of a circumferential wall of the remover body 30. Also, each of the first shield gripping members 40 and second shield gripping members 45 includes a first portion 80 and second portion 81. As illustrated in Fig. 5, the needle shield remover 10 includes first openings 82 that accommodate the second portion 81 and allows the second portion 81 to be flexible. Thus, during assembly the second portion 81 can be pushed and flexed outward by the needle shield in order for the needle shield to enter the remover body 30. Further, the needle shield remover 10 includes second openings 83 between the first portion 80 and the remover body 30. In the present embodiment, the second openings 83 are acting as relieve cuts that allows the first portion 80 to be radially flexible both inwardly and outwardly. The second openings 83 thus provides the first portion 80 with the flexibility that reduces the assembly force required to insert the needle shield into the remover body 30.

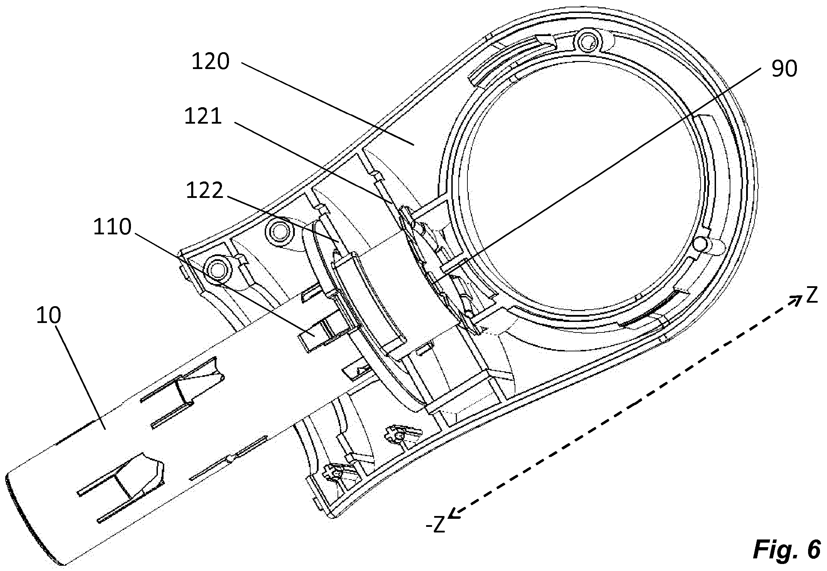

Please now refer to Figs. 2 and 6 for the description on coupling between the needle shield remover 10 and the cap 120, wherein one half of the cap 120 is made transparent to facilitate illustration. As illustrated in Fig. 2, cap fastening members 90 and cut-outs 91 extending radially outward from the proximal end 31 of the remover body 30. During assembly, the cap fastening members 90 are disposed on the first assembly layer 121 of the cap 120, wherein the first assembly layer 121 will catch the cap fastening members 90 and prevent the needle shield remover 10 from moving away from the cap 120 in a -Z direction. On the other hand, the cap 120 further includes a cap attachment member 110 coupled with the cap attachment opening 100 (illustrated in Fig. 2) of the needle shield remover 10. The cap attachment member 110 is configured to rest on the second assembly layer 122 of the cap 120. The second assembly layer 122 will catch the cap attachment member 110 and prevent the needle shield remover 10 from moving toward the cap 120 in a Z direction. In summary, the cap fastening members 90 and the cap attachment member 110 not only couple the needle shield remover 10 with the cap 120 but also prevent the needle shield remover 10 from moving axially with respect to the cap 120.

Further, if the needle shield remover 10 is allowed to rotate axially with respect to the cap 120, its shield gripping members 40, 45 may in turn rotate the needle shield which may cause damages to the needle within the needle shield. Thus, to prevent or at least reduce such rotation, the cut-outs 91 are created in order for the cap fastening members 90 to have acute edges for making contact with the inner surface of the cap 120. In such configuration, if a rotational force is exerted on the needle shield remover 10, the edges of the cap fastening members 90 will collide with the inner surface of the cap 120 to cancel out such rotational force and prevent the needle shield remover 10 from rotating. In Fig. 6, a plurality of cap fastening members 90 are formed at the proximal end 31 of the needle shield remover 10 to prevent the needle shield remover 10 from rotating, but it's not limited thereto. In other embodiment, the proximal end 31 can be configured to have extension of other suitable shapes that maintain contact with the inner surface of the cap 120. Also, the cap attachment member 110 in Fig. 6 is separate from the cap 120. However, in other embodiments, the cap attachment member 110 can be an integral part of the cap 120 for coupling with the cap attachment opening 100 (illustrated in Fig. 2) of the needle shield remover 10 and keep the needle shield remover 10 stable.

In the Figures, various engagement features for are shown for providing an engagement between one or more components of the drug delivery device. The engagement features may be any suitable connecting mechanism such as a snap lock, a snap fit, form fit, a bayonet, lure lock, threads or combination of these designs. Other designs are possible as well.

It should be understood that the illustrated components are intended as an example only. In other example embodiments, fewer components, additional components, and/or alternative components are possible as well. Further, it should be understood that the above described and shown embodiments of the present disclosure are to be regarded as non-limiting examples and that they can be modified within the scope of the claims.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims, along with the full scope of equivalents to which such claims are entitled. It is also to be understood that the terminology used herein is for the purpose of describing particular embodiments only, and is not intended to be limiting.

## Claims

1. A medicament delivery device comprising:
a housing; and
a cap assembly configured to be mounted to the housing to cover a proximal opening of the housing, wherein the cap assembly is arranged on a needle shield; wherein a needle (150) of a syringe (160) is embedded in the needle shield; wherein the syringe (160) is housed in the medicament delivery device; and
wherein the cap assembly includes: a cap; and a needle shield remover (10) comprising
a remover body (30) having a proximal end (31) and a distal end (32); and
at least one first shield gripping member (40), and at least one second shield gripping member (45), wherein the first shield gripping member (40) and the second shield gripping member (45) engage the needle shield;
and wherein the first shield gripping member (40) and the second shield gripping member (45) are arranged offset in relation to each other both in the longitudinal direction and along the circumference surface of the remover body (30); wherein the remover body (30) is substantially cylindrical and has a first circumference (66) and a second circumference (67) circling around different portions of the remover body (30), at least two of the first shield gripping members (40, 45) are distributed around the first circumference (66), at least two of the second shield gripping members (40, 45) are distributed around the second circumference (67);
wherein the first and second shield gripping members (40, 45) are extending radially inwardly and towards the proximal end (31), the first and second shield gripping members (40, 45) engage a circumferential side surface, or the distal end of the needle shield;
wherein the first and second shield gripping members (40, 45) have:
first portions (80) extending from the remover body (30) toward the proximal end (31); and second portions (81) extending radially inward from the first portions (80) and toward the proximal end (31) and engaging the needle shield;
wherein the first and second shield gripping members (40, 45) have first openings (82) between the remover body (30) and first portions (80), the first and second shield gripping members (40, 45) have second openings (83) between the remover body (30) and second portions (81), the first opening (82) is connected to the second opening (83), the first openings (82) allow the first portion (81) to flex radially outward;
**characterized in that** the medicament delivery device further comprises a cap attachment structure that includes a plurality of cap fastening members (90) extending radially outward from the remover body (30), wherein a cut-out (91) exists between two neighbouring cap fastening members (90).

2. The medicament delivery device according to claim 1, wherein only one of the first shield gripping member (40) is placed along a first line, only one of the second shield gripping member (45) is placed along a second line.

3. The medicament delivery device according to claim 1, wherein the second portion of the first shield gripping member has a shape different from that of the second portion of the second shield gripping member.

4. The medicament delivery device according to claim 1, wherein the second portion of the first shield gripping member is shaped substantially identical from the second portion of the second shield gripping member.

5. The medicament delivery device according to claim 1, further comprising at least one cap attachment opening on an outer surface of the remover body 030 to be configured to engage a remover attachment member of the cap.

6. The medicament delivery device according to any one of the preceding claims, wherein the cap assembly includes a remover attachment member placed in the cap and configured to engage the needle shield remover (10).

7. The medicament delivery device according to any one of the preceding claims, wherein the needle shield is a rigid needle shield which covers a flexible needle shield.

## Patentansprüche

1. Medikamentenabgabevorrichtung, umfassend:
ein Gehäuse; und
eine Kappenbaugruppe, die konfiguriert ist, um an dem Gehäuse montiert zu werden, um eine proximale Öffnung des Gehäuses abzudecken, wobei die Kappenbaugruppe an einem Nadelschutz angeordnet ist; wobei eine Nadel (150) einer Spritze (160) in den Nadelschutz eingebettet ist; wobei die Spritze (160) in der Medikamentenabgabevorrichtung untergebracht ist; und
wobei die Kappenbaugruppe einschließt: eine Kappe; und einen Nadelschutzentferner (10), umfassend einen Entfernerkörper (30), der ein proximales Ende (31) und ein distales Ende (32) aufweist; und
mindestens ein erstes Schutzgreifelement (40) und mindestens ein zweites Schutzgreifelement (45), wobei das erste Schutzgreifelement (40) und das zweite Schutzgreifelement (45) den Nadelschutz in Eingriff nehmen;
und wobei das erste Schutzgreifelement (40) und das zweite Schutzgreifelement (45) sowohl in der Längsrichtung als auch entlang der Umfangsoberfläche des Entfernerkörpers (30) versetzt zueinander angeordnet sind;
wobei der Entfernerkörper (30) im Wesentlichen zylindrisch ist und einen ersten Umfang (66) und einen zweiten Umfang (67), die unterschiedliche Abschnitte des Entfernerkörpers (30) umkreisen, aufweist, mindestens zwei der ersten Schutzgreifelemente (40, 45) um den ersten Umfang (66) herum verteilt sind, mindestens zwei der zweiten Schutzgreifelemente (40, 45) um den zweiten Umfang (67) herum verteilt sind;
wobei sich das erste und das zweite Schutzgreifelement (40, 45) radial nach innen und zu dem proximalen Ende (31) hin erstrecken, wobei das erste und das zweite Schutzgreifelement (40, 45) eine Umfangsseitenoberfläche oder das distale Ende des Nadelschutzes in Eingriff nehmen;
wobei das erste und das zweite Schutzgreifelement (40, 45) aufweisen:
erste Abschnitte (80), die sich von dem Entfernerkörper (30) zu dem proximalen Ende (31) hin erstrecken; und zweite Abschnitte (81), die sich von den ersten Abschnitten (80) radial nach innen und zu dem proximalen Ende (31) hin erstrecken und den Nadelschutz in Eingriff nehmen;
wobei das erste und das zweite Schutzgreifelement (40, 45) erste Öffnungen (82) zwischen dem Entfernerkörper (30) und ersten Abschnitten (80) aufweisen, das erste und das zweite Schutzgreifelemente (40, 45) zweite Öffnungen (83) zwischen dem Entfernerkörper (30) und zweiten Abschnitten (81) aufweisen, die erste Öffnung (82) mit der zweiten Öffnung (83) verbunden ist, die ersten Öffnungen (82) es dem ersten Abschnitt (81) ermöglichen, sich radial nach außen zu biegen;
**dadurch gekennzeichnet, dass** die Medikamentenabgabevorrichtung ferner eine Kappenanbringungsstruktur umfasst, die eine Vielzahl von Kappenbefestigungselementen (90), die sich von dem Entfernerkörper (30) radial nach außen erstrecken, einschließt, wobei ein Ausschnitt (91) zwischen zwei benachbarten Kappenbefestigungselementen (90) vorhanden ist.

2. Medikamentenabgabevorrichtung nach Anspruch 1, wobei nur eines des ersten Schutzgreifelements (40) entlang einer ersten Linie platziert ist, nur eines des zweiten Schutzgreifelements (45) entlang einer zweiten Linie platziert ist.

3. Medikamentenabgabevorrichtung nach Anspruch 1, wobei der zweite Abschnitt des ersten Schutzgreifelements eine Form aufweist, die sich von der des zweiten Abschnitts des zweiten Schutzgreifelements unterscheidet.

4. Medikamentenabgabevorrichtung nach Anspruch 1, wobei der zweite Abschnitt des ersten Schutzgreifelements im Wesentlichen identisch zu dem zweiten Abschnitt des zweiten Schutzgreifelements geformt ist.

5. Medikamentenabgabevorrichtung nach Anspruch 1, ferner umfassend mindestens eine Kappenanbringungsöffnung an einer Außenoberfläche des Entfernerkörpers ()30, die konfiguriert werden soll, um ein Entferneranbringungselement der Kappe in Eingriff zu nehmen.

6. Medikamentenabgabevorrichtung nach einem der vorstehenden Ansprüche, wobei die Kappenbaugruppe ein Entferneranbringungselement einschließt, das in der Kappe platziert und konfiguriert ist, um den Nadelschutzentferner (10) in Eingriff zu nehmen.

7. Medikamentenabgabevorrichtung nach einem der vorstehenden Ansprüche, wobei der Nadelschutz ein starrer Nadelschutz ist, der einen flexiblen Nadelschutz abdeckt.

## Revendications

1. Dispositif d'administration de médicament comprenant :
un boîtier ; et
un ensemble capuchon conçu pour être monté au boîtier pour recouvrir une ouverture proximale du boîtier, dans lequel l'ensemble capuchon est arrangé sur un protecteur d'aiguille ; dans lequel une aiguille (150) d'une seringue (160) est logée dans le protecteur d'aiguille ; dans lequel la seringue (160) est logée dans le dispositif d'administration de médicament ; et
dans lequel l'ensemble capuchon comporte : un capuchon ; et un dispositif de retrait de protecteur d'aiguille (10) comprenant un corps de dispositif de retrait (30) présentant une extrémité proximale (31) et une extrémité distale (32) ; et
au moins un premier élément de saisie de protecteur (40), et au moins un second élément de saisie de protecteur (45), dans lequel le premier élément de saisie de protecteur (40) et le second élément de saisie de protecteur (45) viennent en prise avec le protecteur d'aiguille ;
et dans lequel le premier élément de saisie de protecteur (40) et le second élément de saisie de protecteur (45) sont disposés de manière décalée l'un par rapport à l'autre à la fois dans la direction longitudinale et le long de la surface circonférentielle du corps de dispositif de retrait (30) ;
dans lequel le corps de dispositif de retrait (30) est sensiblement cylindrique et présente une première circonférence (66) et une seconde circonférence (67) encerclant différentes parties du corps de dispositif de retrait (30), au moins deux des premiers éléments de saisie de protecteur (40, 45) sont répartis autour de la première circonférence (66), au moins deux des seconds éléments de saisie de protecteur (40, 45) sont répartis autour de la seconde circonférence (67) ;
dans lequel les premier et second éléments de saisie de protecteur (40, 45) s'étendent radialement vers l'intérieur et vers l'extrémité proximale (31), les premier et second éléments de saisie de protecteur (40, 45) viennent en prise avec une surface latérale circonférentielle, ou l'extrémité distale du protecteur d'aiguille ;
dans lequel les premier et second éléments de saisie de protecteur (40, 45) présentent :
des premières portions (80) s'étendant depuis le corps de dispositif de retrait (30) vers l'extrémité proximale (31) ; et des secondes portions (81) s'étendant radialement vers l'intérieur depuis les premières portions (80) et vers l'extrémité proximale (31) et venant en prise avec le protecteur d'aiguille ;
dans lequel les premier et second éléments de saisie de protecteur (40, 45) présentent des premières ouvertures (82) entre le corps de dispositif de retrait (30) et des premières parties (80), les premier et second éléments de saisie de protection (40, 45) présentent des secondes ouvertures (83) entre le corps de dispositif de retrait (30) et des secondes parties (81), la première ouverture (82) est reliée à la seconde ouverture (83), les premières ouvertures (82) permettent à la première partie (81) de fléchir radialement vers l'extérieur ;
**caractérisé en ce que** le dispositif d'administration de médicament comprend en outre une structure d'attache de capuchon qui comporte une pluralité d'éléments de fixation de capuchon (90) s'étendant radialement vers l'extérieur depuis le corps de dispositif de retrait (30), dans lequel une découpe (91) existe entre deux éléments de fixation de capuchon (90) voisins.

2. Dispositif d'administration de médicament selon la revendication 1, dans lequel un seul du premier élément de saisie de protecteur (40) est placé le long d'une première ligne, un seul du second élément de saisie de protecteur (45) est placé le long d'une seconde ligne.

3. Dispositif d'administration de médicament selon la revendication 1, dans lequel la seconde partie du premier élément de saisie de protecteur présente une forme différente de celle de la seconde partie du second élément de saisie de protecteur.

4. Dispositif d'administration de médicament selon la revendication 1, dans lequel la seconde partie du premier élément de saisie de protecteur est de forme sensiblement identique à la seconde partie du second élément de saisie de protecteur.

5. Dispositif d'administration de médicament selon la revendication 1, comprenant en outre au moins une ouverture d'attache de capuchon sur une surface extérieure du corps de dispositif de retrait ()30 pour être conçue pour venir en prise avec un élément d'attache de dispositif de retrait du capuchon.

6. Dispositif d'administration de médicament selon l'une quelconque des revendications précédentes, dans lequel l'ensemble capuchon comporte un élément d'attache de dispositif de retrait placé dans le capuchon et conçu pour venir en prise avec le dispositif de retrait de protecteur d'aiguille (10).

7. Dispositif d'administration de médicament selon l'une quelconque des revendications précédentes, dans lequel le protecteur d'aiguille est un protecteur d'aiguille rigide qui recouvre un protecteur d'aiguille flexible.
